# FASCICULE DE BREVET EUROPEEN

(11) **EP 3 428 141 B1**
(45) Date de publication et mention de la délivrance du brevet: **22.01.2020**
(21) Numéro de dépôt: 18181002.9
(22) Date de dépôt: 29.06.2018
(51) Int. Cl.: C07C 6/12, C07C 7/04, C07C 15/08

(54) **NOUVEAU SCHEMA DE PRODUCTION DE BENZENE A PARTIR DE REFORMAT SANS COLONNE DE TOLUENE**
NEUES HERSTELLUNGSSCHEMA VON BENZOL AUS REFORMAT OHNE TOLUOLSÄULE
NOVEL DIAGRAM FOR PRODUCING BENZENE FROM REFORMATE WITH NO TOLUENE COLUMN

(30) Priorité: 12.07.2017 FR 1756639
(43) Date de publication de la demande: 16.01.2019
(73) Titulaire: IFP Energies nouvelles, 92852 Rueil-Malmaison Cedex (FR)
(72) Inventeur: PAGOT, Alexandre, 69230 ST GENIS LAVAL (FR)
(74) Mandataire: IFP Energies nouvelles

(56) Documents cités:
- US-A- 4 041 091
- US-A- 5 573 645
- US-B1- 6 512 154

## Description

### CONTEXTE DE L'INVENTION

L'invention porte sur le schéma de fractionnement du complexe aromatique. Le complexe aromatique est alimenté par des charges C6 à C10+ et comporte une première étape consistant à éliminer les composés aliphatiques, c'est-à-dire les composés saturés de type paraffiniques.

La deuxième étape permet de fractionner les espèces aromatiques par nombre de carbone avec un enchainement comportant en général 4 colonnes dites colonne de benzène, colonne de toluène, colonne de xylènes, colonne des aromatiques lourds en suivant le nom des produits sortant en tête (sauf pour la colonne des lourds).

Le reformat, les éventuels imports en provenance d'unités situées hors du complexe aromatique, et les flux recyclés en provenance des unités de conversion telles que l'isomérisation, la transalkylation, la dismutation, etc.., alimentent tous ce train de fractionnement à différents niveaux suivant leurs compositions.

L'objet de l'invention est de proposer un schéma de fractionnement du complexe aromatique sans colonne de toluène en adaptant les spécifications, notamment les points de coupes des autres colonnes du complexe pour maintenir un bon fonctionnement et les spécifications des produits.

### EXAMEN DE L'ART ANTERIEUR

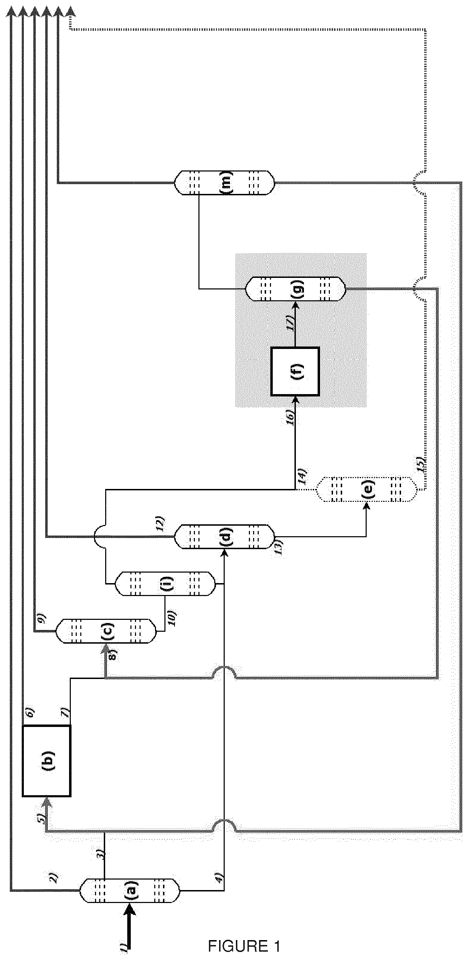

Le complexe aromatique dans sa globalité est généralement représenté selon le schéma de la figure 1 ci-dessous. Toutefois ce schéma ne mentionne pas les colonnes de stabilisation présentes dans les diverses unités de conversions (isomérisation, transalkylation, dismutation, etc...).

Généralement, le toluène est transalkylé ou méthylé pour accroitre la production de paraxylène.

Le contrôle du toluène, c'est-à-dire l'ensemble des actions permettant de maximiser ou de minimiser la production de toluène selon les unités, s'effectue à différents niveaux notés 1, 2, 3 et 4 sur la figure 1 :
1. Le splitter de reformat (a): On maximise la récupération de toluène dans le soutirage latéral (3) afin de l'envoyer à l'unité d'extraction des aromatiques (b). En effet, la coupe C6/C7 des reformats contient encore trop de composés aliphatiques pour être envoyée directement au train de fractionnement. De plus, les effluents des unités catalytiques peuvent contenir du cyclohexane qui ne peut pas être séparé du benzène par distillation. Une partie de la coupe benzène / toluène issue de ces unités doit donc être traitée par l'unité d'extraction des aromatiques.
2. La colonne de benzène (c) produit le benzène, flux (9), aux spécifications de la pétrochimie. La teneur en toluène est alors minimale, ce qui maximise la récupération du toluène au fond de cette colonne, flux (10).
3. Le fond de la colonne de toluène (i) alimente la colonne de xylènes (d) dont le distillat (12) est le produit majoritaire du complexe (C8 aromatiques). Il est nécessaire de contrôler la teneur en toluène dans les C8 aromatiques, flux (12).

Dans cette configuration, l'effluent stabilisé de transalkylation (A7 seul ou A7 et A9/A10) contient des aromatiques de 6 à 10 carbones qu'il est nécessaire de renvoyer directement dans la colonne de benzène pour que cet effluent subissent l'ensemble du fractionnement.

On appelle unité de transalkylation toute unité dont le principe repose sur la transalkylation entre deux aromatiques. Cette définition inclut donc la transalkylation du toluène et de coupe A9/A10, de coupe A9/A10 seules, mais aussi la transalkylation du toluène avec lui-même aussi appelée dismutation ou disproportionation du toluène.

Le brevet US4041091 décrit un fractionnement en aval d'unité de transalkylation ne mentionnant pas explicitement de colonne de toluène, mais la colonne dite « BT column » peut être assimilée à un splitter C7-/C8+ en aval de la transalkylation. Ce brevet revendique l'intégration entre condenseur et rebouilleur de colonne pour un schéma bien précis. L'extraction des aromatiques (type morphylane ou sulpholane) nécessaire à minima à l'extraction du cyclohexane n'est pas mentionnée dans ce brevet, au contraire du procédé selon la présente invention.

Tous les brevets concernant la séparation des aromatiques dans le contexte d'un complexe aromatique font appel à une colonne dite de toluène qui sépare le toluène des xylènes. C'est cette colonne qui est supprimée dans la présente invention avec un gain énergétique qui est évalué dans l'exemple ci-dessous.

### DESCRIPTION SOMMAIRE DES FIGURES

La figure 1 représente le schéma de fractionnement selon l'état de l'art.
La figure 2 représente le schéma de fractionnement selon l'invention.

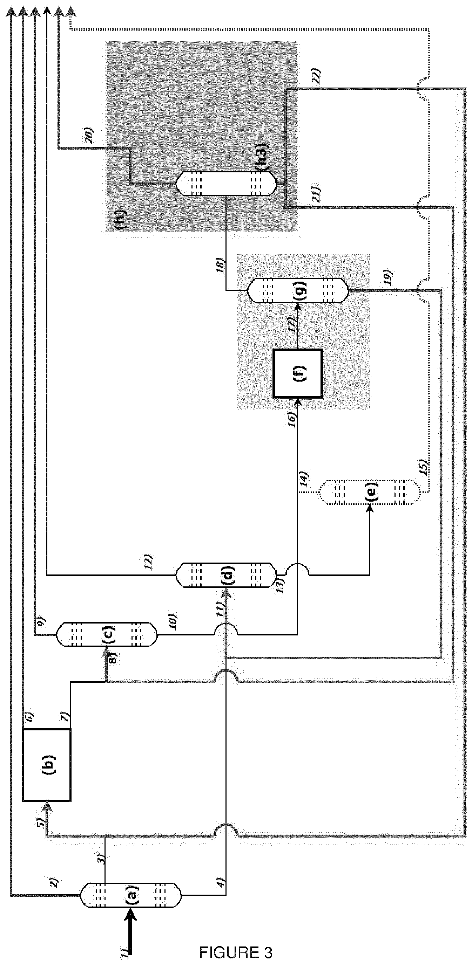

La figure 3 représente le schéma de fractionnement selon l'invention dans une variante où l'unité de stabilisation et de séparation (h) ne comporte qu'une étape notée (h3).
La figure 4 représente le schéma de fractionnement selon l'invention dans une variante où l'unité de stabilisation et de séparation (h) comporte deux étapes successives notées (h1) et (h2).
La figure 5 représente le schéma de fractionnement selon l'invention dans une variante où l'unité de stabilisation et de séparation (h) comporte deux étapes successives notées (h1) et (h2), et le paraxylène (flux 23) est extrait des aromatiques C8, noté flux (12). Le raffinat de cette extraction, flux (24), est converti dans une unité d'isomérisation (k). On parle dans ce cas de « boucle A8 » pour l'extraction du paraxylène, et la conversion des autres isomères avant recycle dans le train de fractionnement.

### DESCRIPTION SOMMAIRE DE L'INVENTION

La présente invention peut se définir comme un procédé de fractionnement faisant partie d'un complexe aromatique dont la charge est généralement un reformat issu d'une unité de reformage catalytique des essences, et dont les produits principaux sont le benzène et les xylènes à un niveau de pureté élevé. Les xylènes peuvent être produits en mélange (Ethyl-benzène, méta-xylène, ortho-xylène, paraxylène) ou par isomère (dans ce cas paraxylène, et éventuellement ortho-xylène). Il est alors nécessaire d'extraire les isomères désirés et de convertir les autres.

Les coproduits du complexe aromatique sont généralement :
- un raffinat issu de l'unité d'extraction des aromatiques, composé d'espèce aliphatique de 6 à 7 atomes de carbones),
- une coupe lourde contenant a minima le naphtalène qui ne peut être valorisée dans le complexe, et qui est un précurseur de coke dans les unités catalytiques,
- Des purges de produits légers incondensables (sections réactionnelles, colonnes de stabilisation).

Plus précisément, le procédé de fractionnement d'un complexe aromatique selon l'invention comprend une unité de séparation du reformat (a), une unité d'extraction des aromatiques (b), une colonne de séparation du benzène (c), une colonne de séparation des xylènes (d), une unité de conversion du toluène par transalkylation (f), avantageusement une unité de séparation des aromatiques lourds (e), une colonne de séparation (g) des effluents issus de l'unité de conversion du toluène (f) et une unité de stabilisation et de séparation des aromatiques légers (h) provenant de l'unité de séparation (g).
a) l'unité de séparation (a) du réformat (1) constituant la charge du procédé permet de séparer :
   - en tête un flux (2) qui est la fraction aliphatique légère de la charge contenant les hydrocarbures à 5 atomes de carbones,
   - latéralement un flux (3), qui est la fraction aromatique légère de la charge, qui est envoyé en mélange avec le flux (22) pour former le flux (5) alimentant l'unité d'extraction des aromatiques (b),
   - et un flux 4), qui est la fraction aromatique lourde de la charge (1) contenant les hydrocarbures aromatiques à plus de 8 atomes de carbones composés majoritairement d'aromatiques.
b) l'unité d'extraction des aromatiques (b) alimentée par le flux (5) produit :
   - le flux (6), raffinat de l'étape d'extraction des aromatiques ne contenant pas d'aromatiques qui est un produit du procédé, et qui est exporté.
   - le flux (7), extrait de l'étape d'extraction des aromatiques, contenant le benzène et le toluène, ce flux (7) est mélangé au flux (21), coupe aromatique légère, stabilisée et recyclée en aval de l'unité d'extraction des aromatiques pour former le flux (8), charge de l'étape de séparation du benzène réalisée dans la colonne (c).
c) l'unité de séparation du benzène (c) permet d'extraire en tête le flux (9) qui est le benzène produit par le complexe, et en fond le flux (10) qui est majoritairement constitué de toluène, envoyé directement à l'unité de transalkylation (f),
   - la fraction aromatique lourde de la charge (1), flux (4), est mélangée à la coupe aromatique lourde (19) extraite en fond de l'étape de séparation (g) des effluents de l'unité de transalkylation (f) pour former le flux (11) qui est la charge de l'unité de séparation des xylènes (d).
d) l'unité de séparation des xylènes (d) permet de séparer en tête un flux (12) ne contenant que des C8 aromatiques, et en fond un flux (13) constitué d'une coupe A9+ qui est, soit exportée du complexe, soit avantageusement traitée dans une étape (e) de séparation des aromatiques lourds.
e) l'unité de séparation des aromatiques lourds (e) permet de récupérer en tête un flux (14) constitué des aromatiques contenants les alkyl-benzènes ayant entre 9 et 10 atomes de carbones, et en fond un flux (15) constitué d'autres hydrocarbures en A10+, non valorisables par les unités de conversion usuelles,
f) l'unité de transalkylation (f) est alimentée par le flux (16) qui résulte de l'addition du flux (10) de fond de la colonne de séparation du benzène (c) et du flux (14), flux de tête de la colonne de séparation des aromatiques lourds (e), et produit un effluent de transalkylation, flux (17), qui est envoyé à l'unité de séparation (g),
g) l'unité de séparation (g) des effluents de l'unité de transalkylation (f) produit en tête un flux (18) d'aromatiques légers qui alimente l'unité de stabilisation et de séparation (h) et en fond le flux (19), coupe aromatique lourde,
h) l'unité (h) de stabilisation et séparation de la coupe aromatique légère alimentée par le flux (18) permet de répartir le recyclage des hydrocarbures à 6 et 7 atomes de carbone constituant le flux (21) qui est envoyé en mélange avec le flux (7) dans l'unité de séparation du benzène (c), et le flux (22) qui est recyclée à l'étape d'extraction des aromatiques (b), ainsi que le flux (20) constituant le gaz de purge. Ce flux (20) est majoritairement constitué des hydrocarbures ayant de 1 à 5 atomes de carbone.

Dans une première variante du procédé selon l'invention, l'unité (h) de stabilisation de la coupe aromatique légère (18) comprend une seule étape (h3) de laquelle est extrait le produit stabilisé qui est réparti en deux flux (21) et (22) en fonction du besoin d'extraction du cyclohexane.

Dans une seconde variante du procédé de fractionnement selon l'invention, l'unité (h) de stabilisation et de séparation de la coupe aromatique légère (18) comprend une première étape (h1) de séparation qui permet de séparer le flux (21) du reste du flux (18), et une seconde étape (h2) dans laquelle rentre le flux résiduel (23), et de laquelle est extrait le flux (22) qui est recyclé vers l'unité d'extraction des aromatiques (b).
Selon une autre variante du procédé de fractionnement d'un complexe aromatique selon l'invention, on implante sur ledit procédé une boucle A8 pour production de paraxylène, ladite boucle A8 étant alimenté par le flux (12) contenant les C8 aromatiques. Les C8 aromatiques sont séparés dans une unité de séparation du paraxylène (j) qui produit le paraxylène, flux (23), et un raffinat, flux (24). Ce raffinat est traité dans une unité de conversion (k) visant à produire du paraxylène à partir des autres isomères. L'effluent (25) est séparé en deux coupes dont la fraction lourde, flux (27), est directement recyclée vers l'unité de séparation des xylènes (d). La fraction légère restante, flux (26) est alors mélangée au flux (18) en provenance de l'unité de transalkylation (f).

Généralement l'extraction du paraxylène dans le cadre du présent procédé est réalisé par un procédé en lit mobile simulé.

Selon une autre variante du procédé de fractionnement d'un complexe aromatique selon la présente invention, on utilise une unité d'isomérisation des xylènes pour désalkyler l'ethylbenzène.

Selon une autre variante du procédé de fractionnement d'un complexe aromatique selon la présente invention, on utilise une unité d'isomérisation des xylènes pour isomériser également l'ethylbenzène en xylènes.
Selon une autre variante du procédé de fractionnement d'un complexe aromatique selon la présente invention, l'unité de transalkylation est une unité de disproportionnation du toluène.

Enfin selon une autre variante du procédé de fractionnement d'un complexe aromatique selon la présente invention, l'unité de transalkylation est une unité de transalkylation du toluène avec une coupe aromatique à 9 et/ou 10 atomes de carbone.

### DESCRIPTION DETAILLEE DE L'INVENTION

L'invention porte sur l'évolution du schéma de fractionnement d'un complexe aromatique.
Dans la suite du texte on utilise souvent la notation A8+ qui signifie l'ensemble des composés aromatiques à 8 atomes de carbone et plus. De la même manière, A9+ désigne l'ensemble des composés aromatiques à plus de 9 atomes de carbone, et ainsi de suite. On appelle aliphatique des composés hydrocarbonés ne contenant pas de noyau aromatique.

Selon le schéma de référence correspondant à l'art antérieur (Cf figure 1), la colonne de toluène sert essentiellement à séparer les A8+ en provenance de la transalkylation du toluène.

La présente invention décrit un procédé de fractionnement du complexe aromatique dans lequel on change le point de coupe de la colonne de fractionnement des effluents de l'unité de transalkylation, et on supprime la colonne de toluène. On soutire en tête de la colonne de stabilisation la coupe aromatique légère constituée de benzène / toluène coupe notée (BT). On contrôle ainsi à la fois la teneur en A8+ dans la coupe BT, et la teneur en toluène dans le produit de fond de la colonne.

La teneur en toluène étant contrôlée, le produit de fond de la colonne peut directement être envoyé à l'unité de séparation des xylènes dont la fonction est de séparer les A8 des A9+. La coupe de tête est une coupe benzène / toluène qui n'est pas stabilisée.

En pratique on cherche à minimiser la quantité de produit à traiter par l'unité d'extraction des aromatiques C6/C7. Il n'est pas nécessaire de traiter l'ensemble de la coupe A6/A7 issue de la transalkylation pour atteindre la pureté en benzène requise. Sur le schéma selon l'art antérieur, cela est ajusté en adaptant la récupération de benzène entre tête / fond de la colonne de stabilisation de la transalkylation.

Dans le nouveau schéma selon l'invention, on scinde en deux la coupe BT (idéalement uniquement le benzène) pour envoyer à l'extraction d'aromatiques uniquement la quantité nécessaire à purifier pour satisfaire la pureté du produit « benzène ».

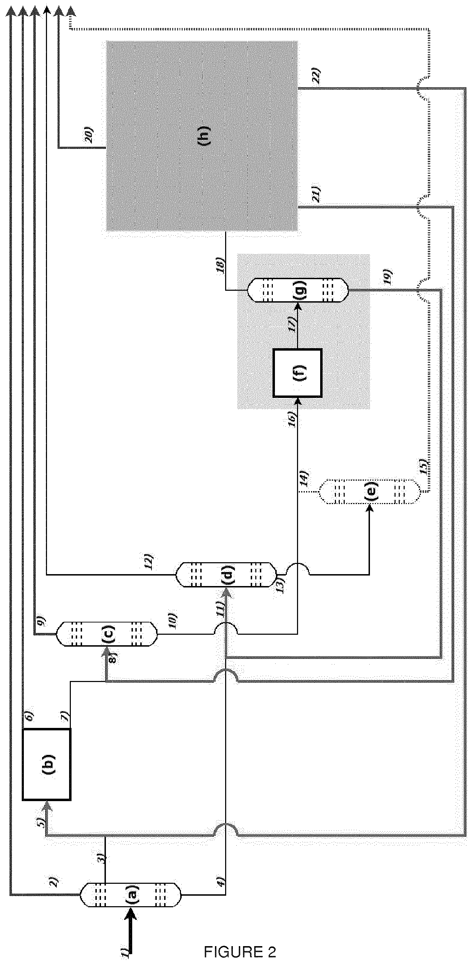

De façon plus précise la description du schéma de procédé selon l'invention peut se faire au moyen de la nomenclature suivante et en suivant le schéma de la figure 2.

Le complexe aromatique selon l'invention comporte les unités suivantes :
(a) unité de séparation du réformat
(b) unité d'extraction des aromatiques
(c) unité de séparation du benzène
(d) unité de séparation des xylènes
(e) unité de séparation des aromatiques lourds (utilisée avantageusement)
(f) unité de transalkylation
(g) unité de séparation des effluents de l'unité de transalkylation
(h) unité de stabilisation et séparation de la coupe aromatique légère extraite à l'étape de séparation des effluents de l'unité de transalkylation

Les flux circulant entre les différentes unités ou communiquant avec l'environnement du procédé (charges ou produits) sont les suivants, en relation avec la figure 2:
1) Charge du complexe aromatique
2) Fraction aliphatique légère de la charge
3) Fraction aromatique légère de la charge
4) Fraction aromatique lourde de la charge
5) Charge de l'étape d'extraction des aromatiques
6) Raffinat de l'étape d'extraction des aromatiques
7) Extrait de l'étape d'extraction des aromatiques
8) Charge de l'étape de séparation du benzène
9) Benzène produit par le complexe
10) Toluène extrait de l'étape de séparation du benzène
11) Charge de l'étape de séparation des xylènes
12) C8 aromatiques
13) Coupe A9+ extraite à l'étape de séparation des xylènes
14) Coupe A9/A10 extraite de l'étape de séparation des aromatiques lourds (dans le cas où une unité (e) de séparation des aromatiques lourds est présente)
15) Coupe A10+ extraite de l'étape de séparation des aromatiques lourds (dans le cas où une unité (e) de séparation des aromatiques lourds est présente)
16) Charge de l'unité de transalkylation
17) Effluents de l'unité de transalkylation
18) Coupe aromatique légère extraite à l'étape de séparation des effluents de l'unité de transalkylation
19) Coupe aromatique lourde extraite à l'étape de séparation des effluents de l'unité de transalkylation
20) Coupe C5- extraite de la coupe aromatique légère
21) Coupe aromatique légère, stabilisée et recyclée en aval de l'unité d'extraction des aromatiques
22) Coupe aromatique légère, stabilisée et recyclée en amont de l'unité d'extraction des aromatiques

Le complexe aromatique est alimenté par la charge du complexe 1). Ce flux riche en aromatiques contient des hydrocarbures contenant de 5 à 11 atomes de carbones et riches en aromatiques. Il s'agit généralement d'un reformat stabilisé. Ce flux est traité à l'étape (a) de séparation du réformat. Le flux 2) est la fraction aliphatique légère de la charge et contient les hydrocarbures à 5 atomes de carbones et ne contient par nature aucun aromatiques. La fraction aliphatique légère de la charge est un coproduit du complexe et sort du complexe aromatiques.

L'étape (a) de séparation du réformat produit le flux 3), fraction aromatique légère de la charge et le flux 4), fraction aromatique lourde de la charge. La fraction aromatique légère de la charge 3) contient les hydrocarbures à 6 et 7 atomes de carbones. Elle est riche en aromatiques mais contient beaucoup de composés aliphatiques. Elle est donc destinée à être traitée dans l'unité (b) d'extraction des aromatiques. La fraction aromatique lourde de la charge 4) contient les hydrocarbures à plus de 8 atomes de carbone composés majoritairement d'aromatiques. Les composés aliphatiques sont présents en très faible quantité et ne nécessite pas une extraction par une unité dédiée.

La fraction aromatique légère de la charge (3) est mélangée au flux (22) qui est la coupe aromatique légère, stabilisée et recyclée en amont de l'unité d'extraction des aromatiques en provenance de l'étape (h) de stabilisation et séparation de la coupe aromatique légère extraite des effluents de l'unité de translation (f). Le flux (5) ainsi obtenu est la charge de l'étape d'extraction des aromatiques (b). Cette unité permet de séparer les composées aliphatiques des composés aromatiques généralement par extraction au solvant. Le flux (6), raffinat de l'étape d'extraction des aromatiques est un autre coproduit du complexe ne contenant pas d'aromatiques il est donc exportés.

Le flux (7), extrait de l'étape d'extraction des aromatiques, contient le benzène et le toluène. Ce flux est mélangé au flux (21), coupe aromatique légère, stabilisée et recyclée en aval de l'unité d'extraction des aromatiques pour former le flux (8), charge de l'étape de séparation du benzène. L'étape de séparation du benzène (c) permet d'extraire le flux (9) qui est le benzène produit par le complexe. Il s'agit généralement d'une colonne de distillation dont le flux (9) est le produit de tête. Le benzène produit par le complexe, flux (9), contient uniquement du benzène aux spécifications de la pétrochimie à laquelle il est destiné. Le reste du flux (8), forme le flux (10) qui est donc le toluène extrait de l'étape de séparation du benzène. Grace à l'arrangement du fractionnement ce flux contient une quantité très faible d'hydrocarbures à plus de 8 atomes de carbone et peut donc directement être envoyé à l'unité (f) de transalkylation.

En parallèle, la fraction aromatique lourde de la charge, flux (4), est mélangée à la coupe aromatique lourde extraite à l'étape de séparation des effluents de l'unité de transalkylation, flux (19), pour former le flux (11) qui est la charge de l'étape de séparation des xylènes (d). Cette étape (d) de séparation des xylènes permet de séparer un flux ne contenant que des C8 aromatiques, flux (12). Ce flux contient donc l'éthyle-benzène et les 3 isomères des xylènes. Suivant la configuration du complexe les C8 aromatiques peuvent être exportés comme produit majoritaire du complexe ou traités dans des unités d'extraction et de conversion pour produire des produits à plus forte valeur ajoutée tel que le para-xylène.

L'étape (d) de séparation des xylènes produit également une coupe A9+ extraite à l'étape de séparation des xylènes, flux 13). Ce flux peut, soit être exporté du complexe aromatique, soit avantageusement être traité dans une étape (e) de séparation des aromatiques lourds. Cette étape permet de récupérer les aromatiques contenants les alkyl-benzènes ayant entre 9 et 10 atomes de carbones dans une coupe A9/A10 extraite de l'étape de séparation des aromatiques lourds, flux (14). Les autres hydrocarbures non valorisables par les unités de conversion connues de l'homme du métier, sont alors purgés et constituent la coupe A10+ extraite de l'étape de séparation des aromatiques lourds, flux (15).

L'unité (f) de transalkylation est alors alimentée par le flux (16) qui provient du mélange du flux (10), toluène extrait de l'étape de séparation du benzène, et avantageusement d'une coupe A9/A10 extraite de l'étape de séparation des aromatiques lourds constituant le flux (14). La charge (16) est convertie par un mécanisme réactionnel de transalkylation qui permet de déplacer les groupes alkyles d'un composé à un autre. L'unité (f) de transalkylation produit des xylènes à partir de molécules à 7, 9 et 10 atomes de carbones. Les effluents de l'unité de transalkylation, flux (17), contiennent généralement des aromatiques de 6 à 10 atomes de carbone, des produits de craquage (méthane, éthane, propanes, etc...), et quelques naphtènes issues d'une réaction parasite d'hydrogénation des cycles aromatiques, notamment du cyclohexane. Si la charge ne contient que du toluène on parle alors de dismutation ou disproportionation du toluène.

Compte tenu des espèces présentent dans les effluents de l'unité de transalkylation (f), le flux (17) doit donc être séparé et stabilisé pour se conformer aux différents produits du complexe.

Selon l'invention, les effluents de l'unité de transalkylation, flux (17), sont dans un premier temps séparés dans l'étape (g) de séparation des effluents de l'unité de transalkylation. La coupe aromatique lourde extraite à l'étape de séparation des effluents de l'unité de transalkylation, flux (19), contient les aromatiques à 8 atomes de carbones et plus. Elle est recyclée vers l'étape (d) de séparation des xylènes.

La coupe aromatique légère extraite à l'étape de séparation des effluents de l'unité de transalkylation, flux (18), contient les aromatiques à 6 et 7 atomes de carbone (benzène et toluène). Elle n'est pas stabilisée, et contient également de naphtènes issues d'une réaction parasite d'hydrogénation des cycles aromatiques, notamment du cyclohexane.

L'étape (h) de stabilisation et séparation de la coupe aromatique légère permet de supprimer les composés à 5 atomes de carbones et moins du flux (18), et de répartir le recyclage de hydrocarbures à 6 et 7 atomes de carbone. En effet le flux (18) contient notamment du cyclohexane qui ne peut être séparé du benzène par distillation du fait d'un écart entre les points d'ébullition de moins de 1°C. De ce fait l'étape (c) de séparation du benzène, ne permet pas de séparer le benzène du cyclohexane. Afin d'assurer les spécifications du benzène produit par le complexe, flux (9), il est nécessaire d'extraire une quantité suffisante de cyclohexane. L'invention permet à l'étape (h) de répartir le recyclage des hydrocarbures à 6 et 7 atomes de carbones. Une quantité est recyclée en entrée de l'unité (b) d'extraction des aromatiques pour extraite le cyclohexane contenu dans le flux (22). Le reste, flux (21), est directement recyclé à l'étape de séparation du benzène (b). Cet arrangement permet de satisfaire les spécifications de pureté du benzène produit par le complexe aromatique, flux (9), tout en minimisant les quantités recyclées vers l'unité d'extraction des aromatiques (b).

Deux modes de réalisation de l'étape (h) de stabilisation et séparation de la coupe aromatique légère sont représentés de de façon non exhaustive et non limitative.

Selon le premier mode de réalisation, le flux (18) est stabilisé dans une étape de stabilisation (h3). Le produit stabilisé est alors simplement réparti en deux flux (21) et (22) en fonction du besoin d'extraction du cyclohexane.

Selon le deuxième mode de réalisation, le flux (18) alimente dans un premier temps une étape (h1) de séparation qui permet de séparer le flux (21) du reste du flux (18). Le flux résiduel (23) n'est alors pas stabilisé, ce qui est fait au travers l'étape (h2) généralement réalisée par une colonne de distillation dite stripper. Le produit stabilisé forme alors le flux (22) qui est recyclé vers l'unité d'extraction des aromatiques (b). Bien que plus complexe, ce mode de réalisation permet de ne pas recycler vers l'unité d'extraction des aromatiques (b) la coupe C7 du flux (18) qui contient majoritairement le toluène.

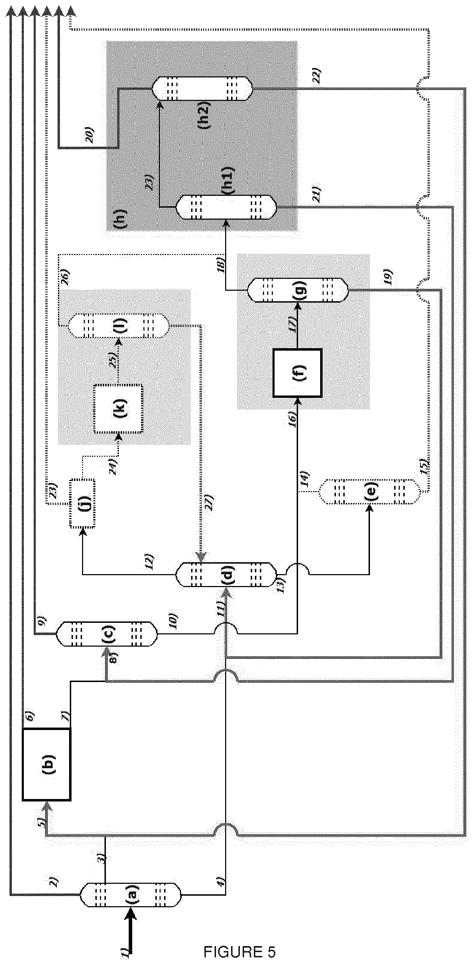

Avantageusement, le schéma de séparation selon l'invention peut être complété afin de produire du paraxylène, flux (23) (Cf. figure 5). Les C8 aromatiques, flux (12), sont alors traités dans une unité (j) d'extraction du paraxylène. Le raffinat, flux (24), de cette unité est composé d'éthyle-benzène, d'ortho-xylène et de méta-xylène. Afin d'augmenter la productivité en paraxylène du complexe, le raffinat est converti dans une unité d'isomérisation (k). Les deux isomères du paraxylène sont alors en partie convertis en paraxylène par isomérisation. L'éthyl-benzène peut soit être désalkylé pour former du benzène, soit également isomérisé pour produire des xylènes. Les effluents, flux (25), sont séparés dans une étape de séparation (l). La fraction lourde composée des C8+, notée flux (27), est recyclée à l'unité d'extraction des xylènes (d), la fraction légère non stabilisée, notée flux (26), est composé majoritairement de benzène et de toluène est mélangé au flux (18), avant d'être traitée dans l'unité (h) de stabilisation et de séparation de la coupe aromatique légère.

### EXEMPLES SELON L'INVENTION

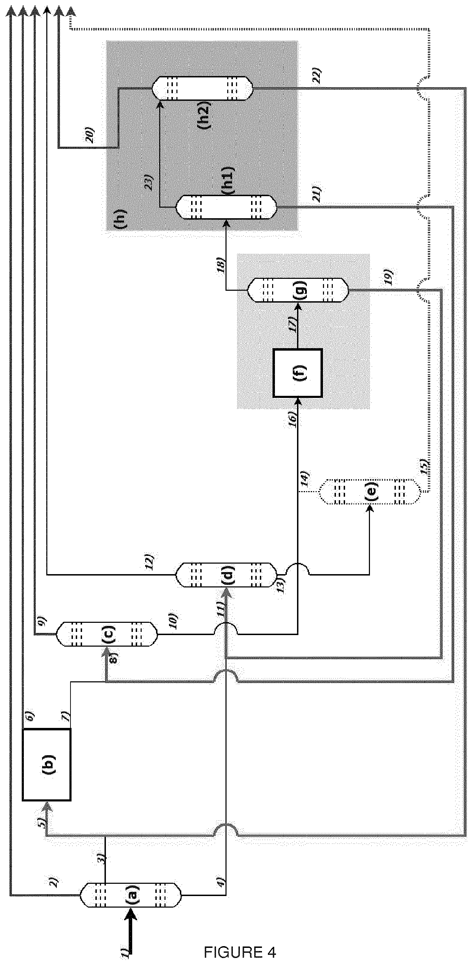

L'exemple compare le schéma de référence selon l'art antérieur (Cf. figure 1) au schéma selon l'invention représenté dans la figure 4.

L'unité (f) de transalkylation est une unité traitant le toluène extrait de l'étape de séparation du benzène, flux (10), et la coupe A9/A10 extraite de l'étape de séparation des aromatiques lourds, flux (14).

Le reformat traité a la composition donnée dans le tableau 1 ci-dessous.

Dans les deux cas la production de paraxylène est de 144,0 t/h, et celle de benzène est de 53,6 t/h pour un débit de reformat de 250 t/h.

**Tableau 1 : composition du reformat en entrée de complexe aromatique (% poids)**

| | |
|---|---|
| C5- | 1,7% |
| C6 non aro | 5,8% |
| C7 non aro | 6,1% |
| C8+ non aro | 0,7% |
| A6 | 6,6% |
| A7 | 24,8% |
| A8 | 30,9% |
| A9 | 17,7% |
| A10 | 4,8% |
| A11 | 0,8% |

L'ensemble des colonnes impactées par le schéma selon l'invention est répertorié dans le Tableau 2 ci-dessous. Le schéma selon l'invention se traduit par la suppression de la colonne de toluène et l'apparition d'une colonne dite de benzène locale, étape (h1). Le gain sur l'énergie de rebouillage cumulée des colonnes dans le schéma selon la présente invention est de 20 MW, pour une valeur de référence de 92,1 MW. Le gain énergétique est donc de 22% dans le cas présenté.

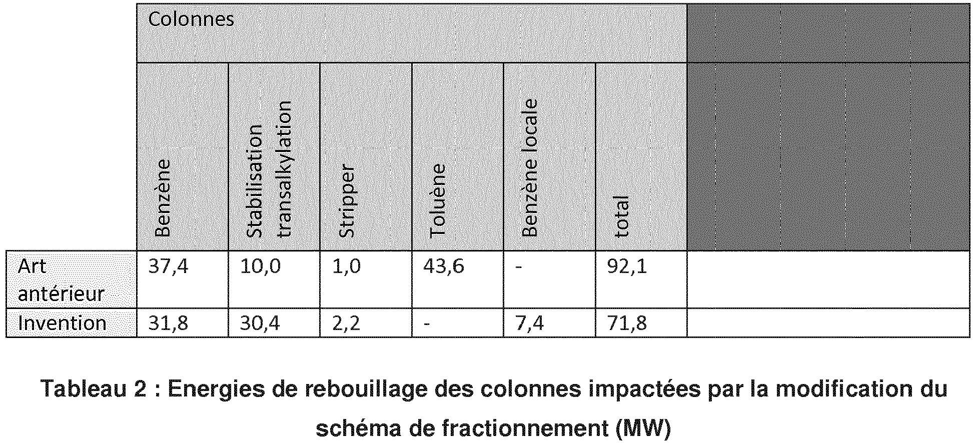

## Revendications

1. Procédé de fractionnement d'un complexe aromatique comprenant une unité de séparation du reformat (a), une unité d'extraction des aromatiques (b), une colonne de séparation du benzène (c), une colonne de séparation des xylènes (d), une unité de conversion du toluène par transalkylation f), une unité de séparation des aromatiques lourds (e), une colonne de séparation (g) des effluents issus de l'unité de conversion du toluène (f) et une unité de stabilisation et de séparation des aromatiques légers (h) provenant de l'unité de séparation (g), procédé dans lequel :
a) l'unité de séparation (a) du réformat (1) constituant la charge du procédé permet de séparer :
• en tête un flux (2) qui est la fraction aliphatique légère de la charge contenant les hydrocarbures à 5 atomes de carbones,
• latéralement un flux (3), qui est la fraction aromatique légère de la charge, qui est envoyé en mélange avec le flux (22) pour former le flux (5) alimentant l'unité d'extraction des aromatiques (b),
• et un flux 4), qui est la fraction aromatique lourde de la charge (1) contenant les hydrocarbures aromatiques à plus de 8 atomes de carbones composés majoritairement d'aromatiques,
b) l'unité d'extraction des aromatiques (b) alimenté par le flux (5) produit
• le flux (6), raffinat de l'étape d'extraction des aromatiques ne contenant pas d'aromatiques qui est un produit du procédé, il est donc exportés.
• le flux (7), extrait de l'étape d'extraction des aromatiques, contenant le benzène et le toluène, ce flux (7) est mélangé au flux (21), coupe aromatique légère, stabilisée et recyclée en aval de l'unité d'extraction des aromatiques pour former le flux (8), charge de l'étape de séparation du benzène réalisée dans la colonne (c),
c) l'unité de séparation du benzène (c) permet d'extraire en tête le flux (9) qui est le benzène produit par le complexe et en fond le flux (10) qui est majoritairement constitué de toluène, envoyé directement à l'unité de transalkylation (f),
• la fraction aromatique lourde de la charge (1), flux (4), est mélangée à la coupe aromatique lourde (19) extraite en fond de l'étape de séparation (g) des effluents de l'unité de transalkylation (f) pour former le flux (11) qui est la charge de l'unité de séparation des xylènes (d),
d) l'unité de séparation des xylènes (d) permet de séparer en tête un flux (12) ne contenant que des C8 aromatiques, et en fond un flux (13) constitué d'une coupe A9+ qui est soit exportée du complexe, soit avantageusement traitée dans une étape (e) de séparation des aromatiques lourds,
e) l'unité de séparation des aromatiques lourds (e) permet de récupérer en tête un flux (14) constitué des aromatiques contenants les alkyl-benzènes ayant entre 9 et 10 atomes de carbones, et en fond un flux (15) constitué d'autres hydrocarbures en A10+, non valorisables par les unités de conversion usuelles,
f) l'unité de transalkylation (f) est alimentée par le flux (16) qui résulte de l'addition du flux (10) de fond de la colonne de séparation du benzène (c) et du flux (14), flux de tête de la colonne de séparation des aromatiques lourds (e), et produit un effluent d'alkylation, flux (17), qui est envoyé à l'unité de séparation (g),
g) l'unité (g) de séparation (g) des effluents de l'unité de transalkylation (f) produit en tête un flux (18) d'aromatiques légers qui alimente l'unité de stabilisation et de séparation (h) et en fond le flux (19),La coupe aromatique lourde extraite à l'étape de séparation des effluents de l'unité de transalkylation, flux (19),
h) l'unité (h) de stabilisation et séparation de la coupe aromatique légère alimentée par le flux (18), permet de répartir le recyclage de hydrocarbures à 6 et 7 atomes de carbone constituant le flux (21) qui est envoyé en mélange avec le flux (7) dans l'unité de séparation du benzène (c), et le flux (22) qui est directement recyclée à l'étape de séparation du benzène (b), ainsi que le flux (20) constituant le gaz de purge majoritairement constitué d'hydrocarbures ayant de 1 à 5 atomes de carbone.

2. Procédé de fractionnement d'un complexe aromatique selon la revendication 1, dans lequel l'unité (h) de stabilisation et de séparation de la coupe aromatique légère (18) comprend une seule étape (h3) de laquelle est extrait le produit stabilisé qui est réparti en deux flux (21) et (22) en fonction du besoin d'extraction du cyclohexane.

3. Procédé de fractionnement d'un complexe aromatique selon la revendication 1, dans lequel l'unité (h) de stabilisation et de séparation de la coupe aromatique légère (18) comprend une première étape (h1) de séparation qui permet de séparer le flux (21) du reste du flux (18) et une seconde étape (h2) dans laquelle rentre le flux résiduel (23) et de laquelle est extrait le flux (22) qui est recyclé vers l'unité d'extraction des aromatiques (b).

4. Procédé de fractionnement d'un complexe aromatique selon les revendications 1 à 3, dans lequel on implante sur ledit procédé une boucle A8 pour production de paraxylène, ladite boucle A8 étant alimenté par le flux (12) contenant les C8 aromatiques. Les C8 aromatiques sont séparés dans une unité de séparation du paraxylène (j) qui produit le paraxylène, flux (23), et un raffinat, flux (24). Ce raffinat est traité dans une unité de conversion (k) visant à produire du paraxylène à partir des autres isomères. L'effluent (25) est séparé en deux coupes dont la fraction lourde, flux (27), est directement recyclée vers l'unité de séparation des xylènes (d). La fraction légère restante, flux (26) est alors mélangée au flux (18) en provenance de l'unité de transalkylation (f).

5. Procédé de fractionnement d'un complexe aromatique selon revendication 4, dans lequel l'extraction du paraxylène est réalisé par un procédé en lit mobile simulé.

6. Procédé de fractionnement d'un complexe aromatique selon la revendication 4, dans lequel on utilise une unité d'isomérisation des xylènes pour désalkyler l'ethylbenzène.

7. Procédé de fractionnement d'un complexe aromatique selon revendication 4, dans lequel on utilise une unité d'isomérisation des xylènes pour isomériser également l'ethylbenzène en xylènes.

8. Procédé de fractionnement d'un complexe aromatique selon la revendication 1, dans lequel l'unité de transalkylation est une unité de disproportionnation du toluène.

9. Procédé de fractionnement d'un complexe aromatique selon revendication 1, dans lequel l'unité de transalkylation est une unité de transalkylation du toluène avec une coupe aromatique à 9 et/ou 10 atomes de carbone.

## Patentansprüche

1. Verfahren zur Fraktionierung eines aromatischen Komplexes, umfassend eine Trennungseinheit des Reformats (a), eine Extraktionseinheit der Aromate (b), eine Trennungssäule des Benzols (c), eine Trennungssäule der Xylole (d), eine Umwandlungssäule des Toluols durch Transalkylation (f), eine Trennungseinheit der schweren Aromate (e), eine Trennungssäule (g) der Abflüsse aus der Umwandlungssäule des Toluols (f) und eine Stabilisierungs- und Trennungseinheit der leichten Aromate (h), die aus der Trennungseinheit (g) stammen, wobei bei dem Verfahren:
a) es die Trennungseinheit (a) des Reformats (1), das die Charge des Verfahrens darstellt, ermöglicht zu trennen:
• am Kopf einen Strom (2), der die aliphatische Fraktion der Charge ist, die die Kohlenwasserstoffe mit 5 Kohlenstoffatomen enthält,
• seitlich einen Strom (3), der die leichte aromatische Fraktion der Charge ist, die im Gemisch mit dem Strom (22) geschickt wird, um den Strom (5) zu bilden, der die Extraktionseinheit der Aromate (b) speist,
• und einen Strom (4), der die schwere aromatische Fraktion der Charge (1), die die aromatischen Kohlenwasserstoffe mit mehr als 8 Kohlenstoffatomen enthält, die mehrheitlich von Aromaten gebildet sind,
b) die Extraktionseinheit der Aromate (b), die mit dem Strom (5) gespeist wird, erzeugt:
• den Strom (6), Raffinat aus dem Extraktionsschritt der Aromate, der keine Aromate enthält, der ein Produkt des Verfahrens ist und somit ausgeschieden wird,
• den Strom (7), Extrakt aus dem Extraktionsschritt der Aromate, der das Benzol und das Toluol enthält, wobei dieser Strom (7) mit dem Strom (21) gemischt wird, leichte aromatische Fraktion, die stromabwärts zur Extraktion der Aromate stabilisiert und rezykliert wird, um den Strom (8) zu bilden, Charge des Trennungsschritts des Benzols, der in der Säule (c) durchgeführt wird,
c) es die Trennungseinheit des Benzols (c) ermöglicht, am Kopf den Strom (9), der das von dem Komplex erzeugte Benzol ist, und am Boden den Strom (10) zu extrahieren, der mehrheitlich von Toluol gebildet ist und direkt an die Transalkylationseinheit (f) geschickt wird
• wobei die schwere aromatische Fraktion der Charge (1), Strom (4), mit der schweren aromatischen Fraktion (19) gemischt wird, die am Boden beim Trennungsschritt (g) der Abflüsse der Transalkylationseinheit (f) extrahiert wird, um den Strom (11) zu bilden, der die Charge der Trennungseinheit der Xylole (d) ist,
d) es die Trennungseinheit der Xylole (d) ermöglicht, am Kopf einen Strom (12), der nur C8 Aromate enthält, und am Boden einen Strom (13) abzutrennen, der von einer A9+ Fraktion gebildet ist, der entweder aus dem Komplex ausgeschieden wird, oder vorteilhafterweise in einem Trennungsschritt (e) der schweren Aromate behandelt wird,
e) es die Trennungseinheit der schweren Aromate (e) ermöglicht, am Kopf einen Strom (14), der von den Aromaten gebildet ist, die die Alkylbenzole mit zwischen 9 und 10 Kohlenstoffatomen enthalten, und am Boden einen Strom (15) wiederzugewinnen, der von anderen Kohlenwasserstoffen bei A10+ gebildet ist, die von den üblichen Umwandlungseinheiten nicht verwertbar sind,
f) die Transalkylationseinheit (f) mit dem Strom (16) gespeist wird, der aus der Hinzufügung des Bodenstroms (10) der Trennungssäule des Benzols (c) und des Stroms (14), Kopfstrom der Trennungssäule der schweren Aromate (e), stammt, und einen Alkylationsabfluss, Strom (17), erzeugt, der an die Trennungseinheit (g) geschickt wird,
g) die Trennungseinheit (g) der Abflüsse der Transalkylationseinheit (f) am Kopf einen Strom (18) von leichten Aromaten, der die Stabilisierungs- und Trennungseinheit (h) speist, und am Boden den Strom (19) erzeugt, schwere aromatische Fraktion, die im Trennungsschritt der Abflüsse der Transalkylationseinheit extrahiert wird, Strom (19),
h) es die Stabilisierungs- und Trennungseinheit (h) der leichten aromatischen Fraktion, die mit dem Strom (18) gespeist wird, ermöglicht, die Rezyklierung von Kohlenwasserstoffen mit 6 und 7 Kohlenstoffatomen, die den Strom (21) darstellen, der im Gemisch mit dem Strom (7) in die Trennungseinheit des Benzols (c) geschickt wird, und den Strom (22), der direkt in dem Trennungsschritt des Benzols (b) rezykliert wird, sowie den Strom (20), der das Spülgas darstellt, das mehrheitlich von Kohlenwasserstoffen mit 1 bis 5 Kohlenstoffatomen gebildet ist, zu verteilen.

2. Verfahren zur Fraktionierung eines aromatischen Komplexes nach Anspruch 1, bei dem die Stabilisierungs- und Trennungseinheit (h) der leichten aromatischen Fraktion (18) einen einzigen Schritt (h3) umfasst, aus dem das stabilisierte Produkt extrahiert wird, das in zwei Ströme (21) und (22) je nach Extraktionsbedarf des Zyklohexans verteilt wird.

3. Verfahren zur Fraktionierung eines aromatischen Komplexes nach Anspruch 1, bei dem die Stabilisierungs- und Trennungseinheit (h) der leichten aromatischen Fraktion (18) einen ersten Trennungsschritt (h1) umfasst, der es ermöglicht, den Strom (21) vom Rest des Stroms (18) zu trennen, und einen zweiten Schritt (h2), in den der Reststrom (23) zurückkommt, und aus dem der Strom (22) extrahiert wird, der zu der Extraktionseinheit der Aromate (b) rezykliert wird.

4. Verfahren zur Fraktionierung eines aromatischen Komplexes nach den Ansprüchen 1 bis 3, bei dem auf dem Verfahren eine Schleife A8 zur Erzeugung von Paraxylol implantiert wird, wobei die Schleife A8 mit dem Strom (12), der die C8 Aromate enthält, gespeist wird, wobei die C8 Aromate in einer Trennungseinheit des Paraxylols (j) getrennt werden, die das Paraxylol, Strom (23) und ein Raffinat, Strom (24) erzeugt, wobei dieses Raffinat in einer Umwandlungseinheit (k) behandelt wird, die Paraxylol aus den anderen Isomeren erzeugen soll, wobei der Abfluss (25) in zwei Fraktionen getrennt wird, eine schwere Fraktion, Strom (27), die direkt zur Trennungseinheit der Xylole (d) rezykliert wird, wobei die restliche leichte Fraktion, Strom (26), nun mit dem Strom (18) aus der Transalkylationseinheit (f) gemischt wird.

5. Verfahren zur Fraktionierung eines aromatischen Komplexes nach Anspruch 4, bei dem die Extraktion des Paraxylols durch ein Verfahren im simulierten Wanderbett durchgeführt wird.

6. Verfahren zur Fraktionierung eines aromatischen Komplexes nach Anspruch 4, bei dem eine Isomerisationseinheit der Xylole verwendet wird, um das Ethylbenzol zu dealkylieren.

7. Verfahren zur Fraktionierung eines aromatischen Komplexes nach Anspruch 4, bei dem eine Isomerisationseinheit der Xylole verwendet wird, um auch das Ethylbenzol in Xylole zu isomerisieren.

8. Verfahren zur Fraktionierung eines aromatischen Komplexes nach Anspruch 1, bei dem die Transalkylationseinheit eine Einheit zur Disproportionierung des Toluols ist.

9. Verfahren zur Fraktionierung eines aromatischen Komplexes nach Anspruch 1, bei dem die Transalkylationseinheit eine Transalkylationseinheit des Toluols mit einer aromatischen Fraktion von 9 und/oder 10 Kohlenstoffatomen ist.

## Claims

1. A fractionation process of an aromatics complex comprising a reformate separation unit (a), an aromatics extraction unit (b), a benzene separation column (c), a xylenes separation column (d), a unit for converting toluene by transalkylation (f), a heavy aromatics separation unit (e), a separation column (g) for effluents obtained from the toluene conversion unit (f) and a stabilization and separation unit (h) for light aromatics originating from the separation unit (g), in which process:
(a) the separation unit (a) for reformate (1) constituting the feed for the process is used in order to separate:
• overhead, a stream (2) which is the light aliphatic fraction of the feed containing hydrocarbons containing 5 carbon atoms,
• as a side stream, a stream (3), which is the light aromatic fraction of the feed, which is sent as a mixture with the stream (22) to form the stream (5) supplying the aromatics extraction unit (b),
• and a stream (4), which is the heavy aromatic fraction of the feed (1), containing aromatic hydrocarbons containing more than 8 carbon atoms composed mainly of aromatics,
(b) the aromatics extraction unit (b) supplied by the stream (5) produces:
• the stream (6), the raffinate from the aromatics extraction step not containing aromatics and which is a product from the process, and which is thus exported,
• the stream (7), the extract from the aromatics extraction step, containing benzene and toluene, this stream (7) being mixed with the stream (21), the light aromatic cut, stabilized and recycled downstream of the aromatics extraction unit in order to form the stream (8), the feed for the benzene separation step carried out in the column (c),
(c) the benzene separation unit (c) is used to extract the overhead stream (9) which is the benzene produced by the complex, and from the bottom, the stream (10) which is mainly constituted by toluene, sent directly to the transalkylation unit (f),
• the heavy aromatic fraction of the feed (1), stream (4), is mixed with the heavy aromatic cut (19) extracted from the bottom of the step (g) for separation of effluents from the transalkylation unit (f) in order to form the stream (11) which is the feed for the xylenes separation unit (d),
(d) the xylenes separation unit (d) is used to separate an overhead stream (12) which contains only C8 aromatics, and from the bottom a stream (13) constituted by an A9+ cut which is either exported from the complex or, advantageously, treated in a step (e) for separating heavy aromatics,
(e) the heavy aromatics separation unit (e) can be used to recover an overhead stream (14) constituted by aromatics containing alkylbenzenes containing 9 to 10 carbon atoms, and from the bottom a stream (15) constituted by other A10+ hydrocarbons which cannot be upgraded by the usual conversion units,
(f) the transalkylation unit (f) is supplied with the stream (16) which results from adding the stream (10) from the bottom of the benzene separation column (c) to the stream (14), the overhead stream from the heavy aromatics separation column (e), and produces an alkylation effluent, stream (17), which is sent to the separation unit (g),
(g) the separation unit (g) for effluents from the transalkylation unit (f) produces an overhead stream (18) of light aromatics which is supplied to the stabilization and separation unit (h), and from the bottom, the stream (19), the heavy aromatic cut extracted at the effluent separation step of the transalkylation unit, stream (19),
(h) the stabilization and separation unit (h) for the light aromatic cut supplied by the stream (18) can be used to divide the recycle of hydrocarbons containing 6 and 7 carbon atoms constituting the stream (21) which is sent to the benzene separation unit (c) as a mixture with the stream (7), and the stream (22) which is recycled directly to the aromatics extraction step (b), as well as the stream (20) constituting the purge gas, mainly constituted by hydrocarbons containing 1 to 5 carbon atoms.

2. The fractionation process of an aromatics complex as claimed in claim 1, in which the unit (h) for stabilization and separation of the light aromatic cut (18) comprises a single step (h3) from which the stabilized product is extracted which is divided into two streams (21) and (22) as a function of the requirement to extract cyclohexane.

3. The fractionation process of an aromatics complex as claimed in claim 1, in which the stabilization and separation unit (h) for the light aromatic cut (18) comprises a first step (h1) for separation which can be used to separate the stream (21) from the rest of the stream (18), and a second step (h2) into which the residual stream (23) returns, and from which the stream (22) is extracted, which is recycled to the aromatics extraction unit (b).

4. The fractionation process of an aromatics complex as claimed in claims 1 to 3, in which a loop A8 for the production of paraxylene is installed in said process, said loop A8 being supplied with the stream (12) containing the C8 aromatics. The C8 aromatics are separated in a paraxylene separation unit (j) which produces paraxylene, stream (23), and a raffinate, stream (24). This raffinate is treated in a conversion unit (k) which is intended to produce paraxylene from other isomers. The effluent (25) is separated into two cuts of which the heavy fraction, stream (27), is recycled directly to the xylenes separation unit (d). The remaining light fraction, stream (26), is then mixed with the stream (18) originating from the transalkylation unit (f).

5. The fractionation process of an aromatics complex as claimed in claim 4, in which the extraction of paraxylene is carried out using a simulated moving bed process.

6. The fractionation process of an aromatics complex as claimed in claim 4, in which a xylenes isomerization unit is employed in order to dealkylate the ethylbenzene.

7. The fractionation process of an aromatics complex as claimed in claim 4, in which a xylenes isomerization unit is also employed in order to isomerize the ethylbenzene into xylenes.

8. The fractionation process of an aromatics complex as claimed in claim 1, in which the transalkylation unit is a toluene disproportionation unit.

9. The fractionation process of an aromatics complex as claimed in claim 1, in which the transalkylation unit is a unit for the transalkylation of toluene with an aromatic cut containing 9 and/or 10 carbon atoms.
